Europäisches Patentamt

European Patent Office

Office européen des brevets

(11) Veröffentlichungsnummer: **0 000 549**
**A1**

(12) # EUROPÄISCHE PATENTANMELDUNG

(21) Anmeldenummer: 78100449.4

(22) Anmeldetag: 20.07.78

(51) Int. Cl.²: **A 61 F 1/00, A 61 C 8/00**

(30) Priorität: 29.07.77 DE 2734249

(43) Veröffentlichungstag der Anmeldung:
07.02.79 Bulletin 79/3

(84) Benannte Vertragsstaaten:
BE CH DE FR GB NL

(71) Anmelder: Bayer Aktiengesellschaft
Zentralbereich Patente,
Marken und Lizenzen Bayerwerk
D-5090 Leverkusen 1. (DE)

(72) Erfinder: Schultz, Peter, Dipl.-Ing.
Schwalbenweg 14
D-5090 Leverkusen. (DE)

(72) Erfinder: Holzrichter, Dieter, Dr.
Susettestrasse 4
D-2000 Hamburg 50. (DE)

(72) Erfinder: Seiler, Hans, Dr.
Wilhelmstasse 16
D-6680 Neunkirchen/Saarland. (DE)

(54) **Endoprothese.**

(57) Die Endoprothese besteht aus einem stielförmigen Implantat das in den Knochen eingesetzt wird wobei das Implantat aus zwei Teilen (1,8) besteht.

Der eine Teil (1) ist als Hohlzylinder ausgebildet, der auf der Innenseite mit axialen Nuten (6) und auf der Aussenseite mit einem Gewinde (4) versehen ist. Das obere Ende des Teiles läuft konisch (3) aus.

Das andere Teil (8) besteht aus einem in den Hohlzylinder formschlüssig einsteckbaren Vollzylinder (8) wenn einer dem Endabschnitt des Hohlzylinders entsprechenden konischen Erweiterung (10). Am axialen Teil befindet sich Stege (12) in Längsrichtung, die in die Nuten (6) des hohlzylindrischen Teiles (1) eingreifen.

FIG. 5

0000549

BAYER AKTIENGESELLSCHAFT     5090 Leverkusen, Bayerwerk
Zentralbereich     Ki/Va
Patente, Marken und Lizenzen

## Endoprothese

Die Erfindung betrifft eine Endoprothese mit einem stielförmigen Implantat, das im Knochen fixiert wird. Die neue
Endoprothese wird in solchen Fällen angewandt, wo es auf
eine genaue äquatoriale Justierung ankommt. Typische Anwendungsfälle sind z.B. der Hüftgelenkersatz, Kniegelenkersatz und Zahnersatz.

Die Problematik der Totalendoprothese z.B. des Hüftgelenkes
ist unter verschiedenen Aspekten zu sehen. Zu den wichtigsten
Aspekten gehört die Langzeitverankerung des Stieles im
Knochen. Es ist bisher üblich, den Stiel mit Zement im
Knochenlager zu verankern. Bei diesen zementverankerten
Prothesen treten nach 5 bis 6 Jahren gehäuft Lockerungen
auf. Um auch bei jüngeren Menschen, deren Lebenserwartung
über der Haltbarkeit der bisherigen Prothesen und Prothesenverankerungen liegt, einen prothetischen Hüftgelenkersatz
durchführen zu können, werden in jüngster Zeit auch unterschiedlichst profilierte Stiele erprobt. Diese werden mit
einem bisher nicht vermeidbaren Spiel im Knochen eingepaßt.
Durch längere Entlastung des Beines werden Bewegungen des
Implantates gering gehalten, um so eine knöcherne Ummauerung
und Fixierung des Prothesenstieles zu erreichen. Durch das
bisher nicht vermeidbare Spiel kann darüber hinaus

Le A 16 845-Ausland

die gelenkgerechte Stellung des Implantates nicht gewährleistet werden.

Die bisherigen Verankerungsarten bringen folgende Nachteile mit sich:

1. Bei sofortiger Belastbarkeit zementverankerter Prothesen treten ungünstige Wechselwirkungen mit dem Gewebe auf.

2. Zementfreie Verankerungen lassen bislang kaum die Möglichkeit des exakten, gelenkgerechten Ausrichtens zu. Das nicht vermeidbare Spiel des Implantates ermöglicht Wackelbewegungen. Dadurch bildet sich ein minderwertiges Fixationsgewebe.

Der Erfindung liegt daher die Aufgabe zugrunde, ein Implantat zu entwickeln, das zementlos und gelenkgerecht eingesetzt werden kann und sofort belastbar ist.

Diese Aufgabe wird erfindungsgemäß dadurch gelöst, daß das Implantat aus zwei Teilen besteht und das eine Teil als Hohlzylinder ausgebildet ist, der auf der Innenseite mit axialen Nuten und auf der Außenseite mit einem Gewinde versehen ist und am oberen Ende einen konischen Endabschnitt aufweist und daß das andere Teil als ein in den Hohlzylinder formschlüssig einsetzbarer Vollzylinder mit in die Nuten eingreifenden axialen Stegen und einer dem Endabschnitt entsprechenden konischen Erweiterung ausgebildet ist. Mittels des Außengewindes läßt sich der Hohlzylinder zementfrei und ohne Spiel bei sofortiger Belastbarkeit im Knochen verankern. Der Nuten-Steg-Formschluß ermöglicht eine gelenkgerechte äquatoriale radiale Ausrichtung des Prothesenkopfes am oberen Ende des Vollzylinders. Durch die konische Ausbildung von Hohl- und Vollzylinder am oberen Ende wird eine Verklemmung des eingeschraubten Hohlzylinders bei Erreichen der gewünschten Endstellung bewirkt und eine Rotationsstabilität der Prothese erreicht.

Le A 16 845-Ausland

Vorteilhaft sind auch auf der Außenseite des Hohlzylinders axiale Nuten mit einer Breite von 1 bis 8 mm eingefräst. Darüber hinaus ist es günstig, wenn die äußere Oberfläche des Hohlzylinders aufgerauht ist.

Im Laufe der Zeit kann das Knochengewebe in diese äußeren Nuten einwachsen, was eine zusätzliche Rotationsstabilisierung bewirkt.

Mit der Erfindung werden folgende Vorteile erzielt:

1. Durch die Zweiteilung der Prothese läßt sich der Kopfteil unabhängig von der Endstellung des festgeklemmten Hohlzylinders gelenkgerecht, radial ausgerichtet fixieren.

2. Mittels des Gewindes läßt sich das Implantat spielfrei und damit ohne Beeinträchtigung des Fixationsgewebes verankern.

3. Das Implantat kann zementlos eingesetzt werden. Wechselwirkungen mit dem Gewebe werden ausgeschaltet.

4. Die äußeren Nuten des Hohlzylinders bewirken neben dem in Endstellung verklemmenden oberen konischen Teil bei bereits fest verankerter Prothese durch einwachsendes Fixationsgewebe eine zusätzliche Rotationsstabilität.

5. Die Verankerung durch ein Gewinde vergrößert die Oberfläche des Implantates und verbessert dadurch die Haftung.

6. Die spielfreie, gelenkgerechte Verankerung erweitert die Anwendungsmöglichkeit von isoelastischen Werkstoffen.

Im folgenden wird die Erfindung am Beispiel einer in der Zeichnung dargestellten Oberschenkelhalsprothese näher erläutert.

Es zeigen:

Figur 1 eine Draufsicht (senkrecht zur Achse des Außenteiles)

Le A 16 845-Ausland

0000549

Figur 2    einen vergrößerten Querschnitt A-A gemäß Fig. 1,
Figur 3    eine Draufsicht des vollzylindrischen Innenteiles,
Figur 4    einen vergrößerten Querschnitt B-B gemäß Fig. 3,
Figur 5    eine modifizierte Ausführung, bei der die Nuten zur
           radialen Fixierung an dem konischen Endabschnitt
           des hohlzylindrischen Außenteiles angebracht sind.

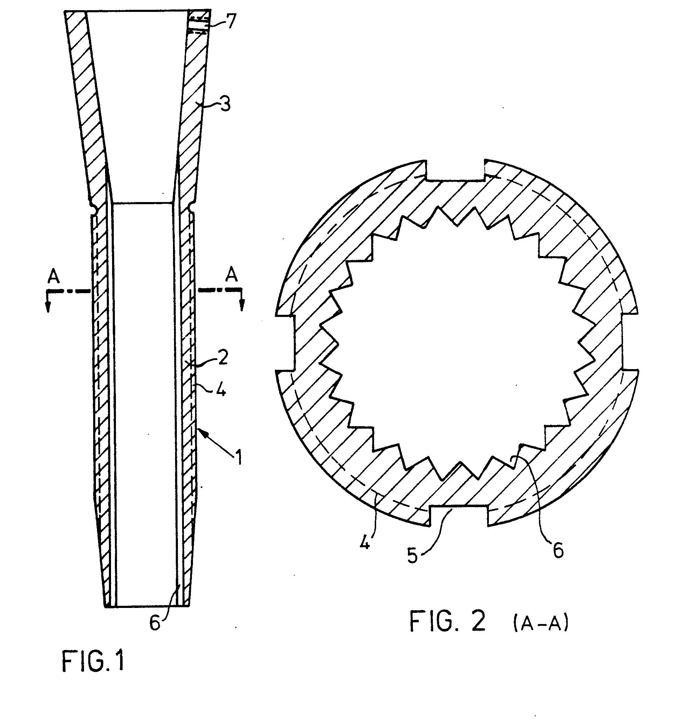

Das hohlzylindrische Außenteil 1 (Gewindehülse gemäß Fig. 1)
besteht aus der geraden Hülse 2 und dem konischen Endabschnitt
3. Im Bereich der Hülse 2 ist ein Gewinde 4 (s. Fig. 1 und 2),
z.B. M 18 x 1,5 eingeschnitten. Außerdem sind auf der Außenseite mehrere axiale Nuten 5 mit einer Breite zwischen 1 bis
8 mm eingefräst. Die Tiefe der Nuten 5 entspricht der Gewindetiefe (u.U. auch tiefer), so daß der Gewindesteg durch die
Nuten vollständig unterbrochen wird. Die Innenfläche der Hülse
2 ist mit einer Vielzahl gleichmäßig über den Umfang verteilter
dreieckförmiger Nuten 6 versehen (z.B. 24 Zähne mit einem
Modul von 0,5 mm).

Der obere Endabschnitt 3 des Hohlzylinders 1 ist konisch erweitert und besitzt am oberen Rand Gewinde 7 für Arretierschrauben.

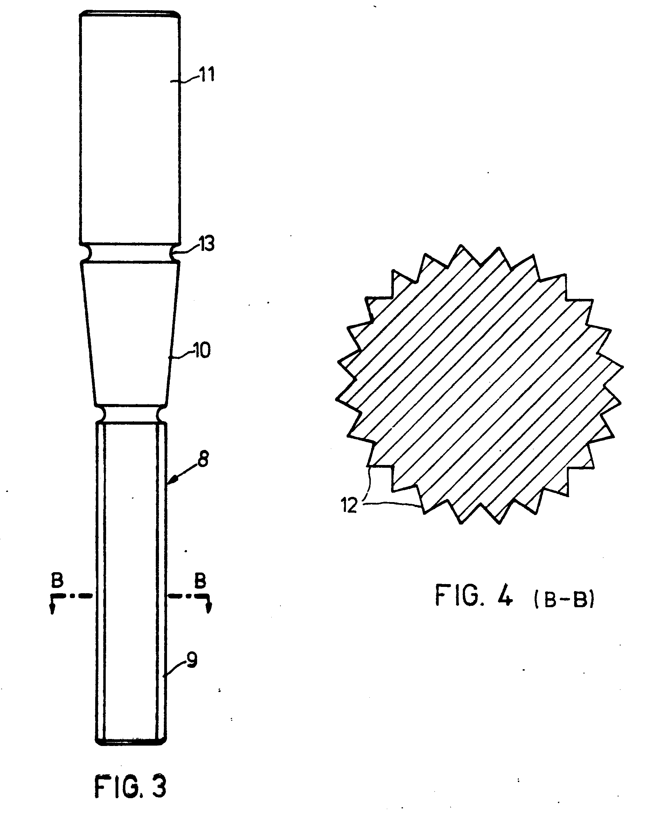

Das vollzylindrische Innenteil 8 gemäß Fig. 3 und 4 besteht
aus dem zylindrischen Stiel 9, der konischen Erweiterung 10
und dem Kopfansatz 11. Der Stiel 9 ist mit axialen, gleichmäßig über den Umfang verteilten dreieckigen Stegen 12
(s.Fig. 4) versehen. Die Stege 12 sind so geformt, daß sie
beim Zusammenbau von Innenteil 8 und Außenteil 1 formschlüssig
in die dreieckförmigen Nuten 6 eingreifen. Auf diese Weise
ist das Innenteil 8 in zusammengebautem Zustand radial fixiert.
Die konische Erweiterung 10 hat die gleiche Steigung wie der
konische Endabschnitt 3 des Außenteiles. Die in das Gewinde 7
(Fig. 1) eingeschraubten Imbusschrauben greifen in die Ringnut 13 (Fig. 3) und sorgen für einen Preßsitz des Innenteiles

Le A 16 845

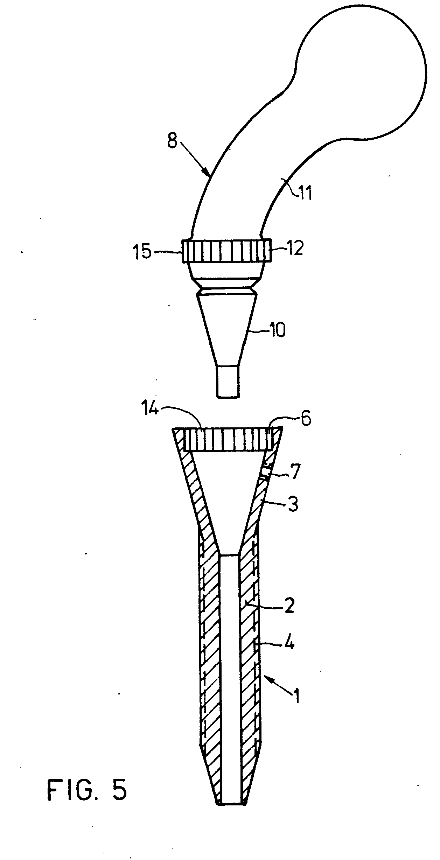

9 im Außenteil 1. Der Kopfansatz 11 ist in üblicher Weise (s. Fig. 5) gekrümmt ausgebildet. Die Außenfläche der Hülse 2 ist aufgerauht, um ein besseres Anwachsen des Kallus zu ermöglichen. Sämtliche Teile sind aus einem Edelstahl für medizinische Anforderungen gefertigt. Eine Fertigung mit einem Emailüberzug ist ebenfalls möglich.

Fig. 5 zeigt eine modifizierte Ausführung. Der Endabschnitt 3 des Hohlzylinders 1 geht hier in eine zylindrische Öffnung 14 über. Die Nuten 6 zur radialen Fixierung des vollzylindrischen Innenteiles 8 sind längs der Mantelfläche dieser zylindrischen Öffnung 14 eingefräst. Der übrige Teil des Hohlzylinders 1 trägt keine Nuten. In entsprechender Weise ist das vollzylindrische Innenteil auf einem kurzen Stück 15 mit axialen Stegen 12 versehen, die formschlüssig in die Nuten 6 eingreifen. Diese Ausführung hat sich vor allem dann bewährt, wenn man zu kleineren Dimensionen übergehen will (Reduzierung von Durchmesser und Länge des hohlzylindrischen Außenteiles).

Zum Einsetzen des zweiteiligen Implantates wird in den Knochen ein dem Gewinde 4 entsprechendes Muttergewinde geschnitten. Dann wird das Außenteil 1 eingeschraubt. Anschließend wird das Innenteil 8 eingesetzt und mittels der in die Gewinde 7 eingeschraubten Imbusschrauben fixiert. Die radiale Feinverzahnung 6, 12 ermöglicht eine sehr genaue Justierung in radialer Richtung. Aufgrund der exakten Justiermöglichkeit, der stabilen Fixierung und der großen Oberfläche der Hülse 2 ist das neue Implantat allen bisher eingesetzten Prothesen überlegen.

Le A 16 845

Wird die Prothesenkombination bei einem Kniegelenkersatz benutzt, so wird der hohlzylindrische Außenteil 1, wie oben beschrieben, im Schienbeinknochen verankert. Das Innenteil 8 trägt dann an Stelle des Kopfansatzes 11 eine Bodenplatte mit Abrollflächen für die gelenkförmigen Tragflächen des im Oberschenkel fixierten Prothesenteiles. Eine genauere Beschreibung einer Kniegelenk - Endoprothese findet sich z.B. in der DE-OS 22 44 064. Im Rahmen dieser Anmeldung soll lediglich auf die zweiteilige Ausbildung des im Unter- bzw. Oberschenkel verankerbaren Teiles hingewiesen werden, die eine genaue äquatorialle Justierung der Gelenkteile ermöglicht.

Eine weitere Anwendung ist der künstliche Zahnersatz. In diesem Fall wird zunächst nur der hohlzylindrische Teil (etwa gemäß Fig. 1) im Kiefer fixiert. Der einsteckbare vollzylindrische Teil trägt den künstlichen Zahn und wird erst dann im Hohlzylinder fixiert, wenn dieser fest im Kiefer eingewachsen ist( in der Regel nach ca. 2 Wochen). Dies hat den Vorteil, daß der im Kiefer fixierte Teil störungsfrei einwachsen kann und nicht durch vorzeitige Belastungen z.B. beim Kauen in seiner Lage verändert wird. Davon abgesehen ist die äquatoriale Justierung des Zahnersatzes im Gegensatz zur einteiligen Zahnprothese unproblematisch.

0000549

### Patentansprüche:

1.      Endoprothese bestehend aus einem stielförmigen Implantat, das in den Knochen eingesetzt wird, dadurch gekennzeichnet, daß das Implantat aus zwei Teilen besteht und das eine Teil als Hohlzylinder (1,2) ausgebildet ist, der auf der Innenseite mit axialen Nuten (6) und auf der Außenseite mit einem Gewinde (4) versehen ist und am oberen Ende einen konischen Endabschnitt (3) aufweist, und daß das andere Teil als ein in den Hohlzylinder (1) formschlüssig einsteckbarer Vollzylinder (8) mit in die Nuten eingreifenden axialen Stegen (12) und einer dem Endabschnitt entsprechenden konischen Erweiterung (10) ausgebildet ist.

2.      Endoprothese nach Anspruch 1, dadurch gekennzeichnet, daß auf der Außenseite des Hohlzylinders (2) axiale Nuten mit einer Breite von 1 bis 8 mm eingefräst sind.

3.      Endoprothese nach Anspruch 1 bis 2, dadurch gekennzeichnet, daß die äußere Oberfläche des Hohlzylinders (1) aufgerauht ist.

4.      Endoprothese nach Anspruch 1 bis 3, dadurch gekennzeichnet, daß der konische Endabschnitt (3) des Hohlzylinders (1) mit einer zylindrischen Öffnung (14) abgeschlossen ist, längs deren Mantelfläche die axialen Nuten (6) angeordnet sind.

<u>Le A 16 845-Ausland</u>

FIG.1

FIG. 2 (A-A)

11

13

10

8

B    B

9

FIG. 3

12

FIG. 4 (B-B)

0000549

FIG. 5

Europäisches
Patentamt

| | EINSCHLÄGIGE DOKUMENTE | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | A 61 F 1/00<br>A 61 C 8/00 |
| X | <u>FR - A - 2 295 729</u> (MAHAY)<br><br>* Seite 2, Zeile 39 - Seite 3, Zeile 10; Seite 7; Patentanspruch 9 *<br><br>-- | 1,4 | |
| PE | <u>DE - A - 2 621 666</u> (REIMER,LYSELL)<br><br>* Seite 1, Absätze 1-4; Ansprüche 1-4 *<br><br>-- | 1,4 | |
| PE | <u>FR - A - 2 366 005</u> (MAHAY)<br><br>* Seite 1, Zeile 40 - Seite 2, Zeile 12; Seite 4, Zeilen 1-14; Anspruch 1 *<br><br>-- | 1 | RECHERCHIERTE SACHGEBIETE (Int. Cl.²)<br><br>A 61 F 1/00<br>A 61 C 8/00 |
| | <u>CH - A - 543 881</u> (MATHYS)<br><br>* Spalte 1, Zeilen 37-42 *<br><br>-- | 2,4 | |
| A | <u>DE - A - 2 318 396</u> (FELDMUEHLE)<br><br>* Seite 7, Absätze 4-7; Seite 10, Absatz 4 - Seite 11, Absatz 2; Ansprüche 8-13 *<br><br>-- | 1 | |
| A | <u>DE - A - 2 340 546</u> (PFAUDLER)<br><br>* Seite 17; Absätze 2-3; Figur 9 *<br><br>---- | 1 | |

KATEGORIE DER GENANNTEN DOKUMENT

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patent- familie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 24-10-1978 | PESCHEK |

EPA form 1503.1 06.78